# EUROPEAN PATENT APPLICATION

(11) **EP 3 721 812 A1**
(43) Date of publication of application: **14.10.2020**
(21) Application number: 20168907.2
(22) Date of filing: 09.04.2020
(51) Int. Cl.: A61B 17/00, A61B 17/04, A61B 17/11

(54) **PURSE STRING SUTURE DEVICE**

(30) Priority: 11.04.2019 US 201962832304 P; 12.03.2020 US 202016816988
(71) Applicant: Covidien LP, Mansfield, MA 02048 (US)
(72) Inventor: AVVLN, Srinivasa Murthy Aravalli, 500049 Hyderabad (IN); GUTTI, Ravi Sekhar, 500049 Hyderabad (IN); MANDULA, Rajanikanth, 500040 Hyderabad (IN)
(74) Representative: Maschio, Antonio

(57) **Abstract**

A suture device is configured to form a purse string suture in procedures such as, e.g., transanal total mesorectal excision (TaTME), for removal of low rectal and ultra-low rectal tumors and preservation of anal sphincters to avoid permanent stomas. The suture device may provide uniform needle rotation, tissue penetration, and/or suture advancement independent of the skill of the clinician. The suture device includes a plurality of needles operatively associated with a needle driver such that rotation of the needle driver transitions the plurality of needles between a retracted position, in which, the plurality of needles extends radially inward, and an extended position, in which, the plurality of needles rotates to extend radially outward to engage and pierce through tissue to form a purse string suture.

## Description

### CROSS-REFERENCE TO RELATED APPLICATIONS

This application claims the benefit of and priority to U.S. Provisional Patent Application Serial No. 62/832,304, filed April 11, 2019, the entire disclosure of which is incorporated by reference herein.

### BACKGROUND

### Technical Field

This disclosure relates to a surgical device and, more particularly, to a surgical device for forming a purse string suture using a plurality of retractable needles and a suture.

### Background of Related Art

Purse string suture devices may include a pair of serrated tissue clamping jaws provided with teeth for clamping the tissue to be sutured therebetween. Such devices include needle passages which extend through the teeth on each jaw for receiving a needle attached to a suture to be threaded through the tissue. In use, the tissue to be sutured is clamped between the jaws and the needle is manually passed through the needle passages in both jaws to thread the suture through the tissue. Thereafter, the jaws are opened and the purse string suture is tightened and wrapped to draw the tissue together. With this type of device, a considerable amount of manual effort and dexterity is required to accomplish the purse string suturing technique. Also, in such devices, it is difficult to control the flow of tissue between the teeth because an insufficient amount of space is provided to gather the tissue clamped by the jaws.

### SUMMARY

In accordance with the disclosure, a suture device includes an outer sleeve defining a lumen therethrough, a needle driver, an inner shaft, and a needle assembly. The needle driver includes a stem extending through the lumen of the outer sleeve, and a wing extending radially outward from the stem. The inner shaft includes an elongate member and a support coupled to a distal end portion of the elongate member. The needle assembly includes a needle and a suture. The needle is rotatably supported on the support of the inner shaft such that the needle is transitionble between a retracted position, in which, the needle is disposed radially inward of a peripheral portion of the outer sleeve, and an extended position, in which, the needle extends radially outward from the peripheral portion of the outer sleeve.

In an embodiment, the outer sleeve may have a frusto-conical configuration.

In another embodiment, the needle may have an arcuate profile.

In yet another embodiment, the needle may have a first end having a tip configured to penetrate tissue, and a second end operatively supported on the support of the inner shaft to enable rotation thereof.

In still yet another embodiment, the second end of the needle may have a boss configured to be received in a groove defined in the support of the inner shaft.

In still yet another embodiment, the second end of the needle may define a bore dimensioned to receive the suture therethrough.

In an embodiment, the support may define the groove adjacent a peripheral portion of the support.

In another embodiment, the outer sleeve may define an aperture at a proximal end portion thereof. The aperture may be in communication with the lumen of the outer sleeve.

In yet another embodiment, the support of the inner shaft may include a guide protrusion configured to be received in a groove defined in the outer sleeve to facilitate axial displacement of the inner shaft relative to the outer sleeve.

In still yet another embodiment, the outer sleeve, the needle driver, and the inner shaft may be concentrically disposed.

In an embodiment, a proximal end portion of the needle driver may extend proximally from a proximal end portion of the outer sleeve, and a proximal end portion of the inner shaft may extend proximally from the proximal end portion of the needle driver.

In accordance with another embodiment, a suture device includes an outer sleeve defining a lumen therethrough, a needle driver, a suture assembly, and an inner shaft. The needle driver includes a stem and wings extending radially outward from the stem. The suture assembly includes needles and a suture. The inner shaft includes a support configured to support the needles thereon. The needles are separated by the wings of the needle driver and rotatably supported on the support of the inner shaft, such that rotation of the needle driver transitions the needles from a retracted position, in which, the needles are disposed flush with a peripheral portion of the outer sleeve or radially inward of the peripheral portion of the outer sleeve, and an extended position, in which, the needles extend radially outward from the peripheral portion of the outer sleeve to enable penetration through tissue.

In an embodiment, the outer sleeve may have a tapered configuration.

In accordance with yet another embodiment, a suture device includes a suture assembly, an outer sleeve, a needle driver, and an inner shaft. The suture assembly includes needles and a suture extending through needles. The needle driver includes a stem and wings configured for concomitant rotation with the stem. The inner shaft includes a support configured to support the needles of the suture assembly between the support and the outer sleeve. The needle driver is rotatable to transition the needles between a retracted position, in which, the needles are disposed radially inward of the support of the inner shaft and the outer sleeve, and an extended position, in which, the needles are disposed radially outward of the support of the inner shaft and the outer sleeve to engage tissue. The inner shaft is movable towards and away from the outer sleeve to receive the needles therebetween.

### BRIEF DESCRIPTION OF THE DRAWINGS

Various embodiments of the disclosure are described hereinbelow with reference to the drawings, wherein:
FIG. 1 is a side view of a suture device in accordance with this disclosure;
FIG. 2 is a side view of the suture device of FIG. 1, illustrating the suture device in a releasing position without a suture assembly;
FIG. 3 is a perspective view of the suture device of FIG. 1 with parts separated;
FIG. 4 is a bottom perspective view of the suture device of FIG. 1, with an inner shaft removed; and
FIG. 5 is a perspective view of the suture device of FIG. 1, illustrating a plurality of needles in a radially extended position.

### DETAILED DESCRIPTION

This disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the disclosure in unnecessary detail.

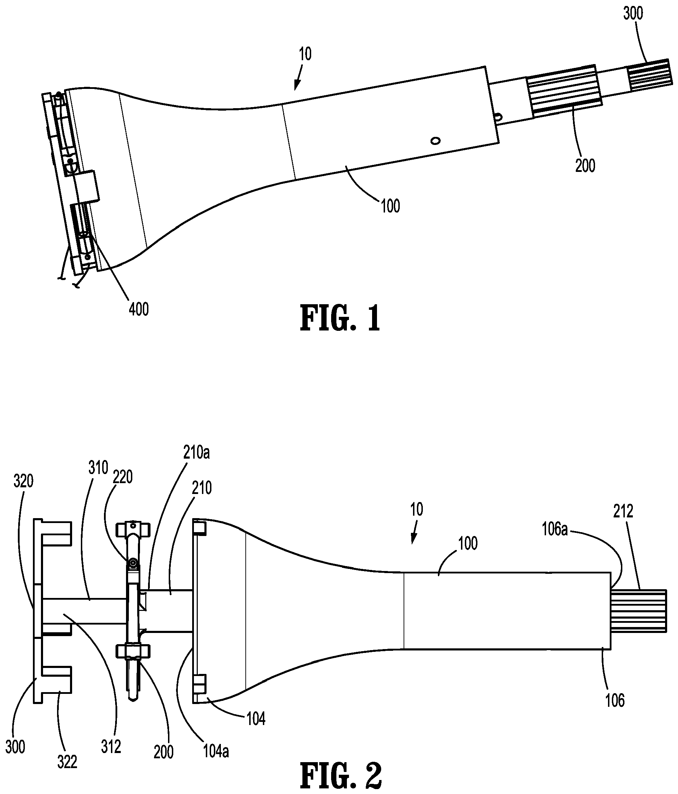

Referring to FIG. 1, there is illustrated a suture device 10 in accordance with this disclosure. The suture device 10 is configured to form a purse string suture. For example, the suture device 10 may be utilized in transanal total mesorectal excision (TaTME) for removal of low rectal and ultra-low rectal tumors and preservation of anal sphincters to avoid permanent stomas. In particular, the suture device 10 may be utilized in forming surgical stitches used to close internal anal structure or to narrow a passage for performing further transanal dissection to create total mesorectal excision.

Through the use of the suture device 10, the formation of a purse string suture is simplified by, e.g., eliminating the need for maneuvering a needle inside an anal canal. In addition, uniform needle rotation, tissue penetration, and/or suture advancement may be obtained independent of the skill of the clinician. In this manner, injuries to tissue may be reduced.

FIGS. 1-3 illustrate the suture device 10 generally including an outer sleeve 100 defining a lumen 102 therethrough, a needle driver 200 rotatably extending through the lumen 102 of the outer sleeve 100, an inner shaft 300 extending through the needle driver 200 for axial displacement therein, and a needle assembly 400 operatively associated with the needle driver 200. The suture device 10 may be configured as a single-use device that is discarded after use or returned to a manufacturer for reprocessing, as a reusable device capable of being cleaned and/or sterilized for repeated use by the end-user, or as a partially-single-use, partially-reusable device. With respect to partially-single-use, partially-reusable configurations, the outer sleeve 100, the needle driver 200, and/or the inner shaft 300 may be configured as a cleanable/sterilizable, reusable component, while the needle assembly 400 is configured as a single-use, disposable/reprocessable component. In either of the above configurations, the outer sleeve 100, the needle driver 200, the inner shaft 300, and the needle assembly 400 are configured to releasably engage each other to facilitate disposal/reprocessing of any single-use components and cleaning and/or sterilization of any reusable component. Further enabling releasable engagement of the components allows for use of different needle assemblies and/or needle drivers with the outer sleeve 100.

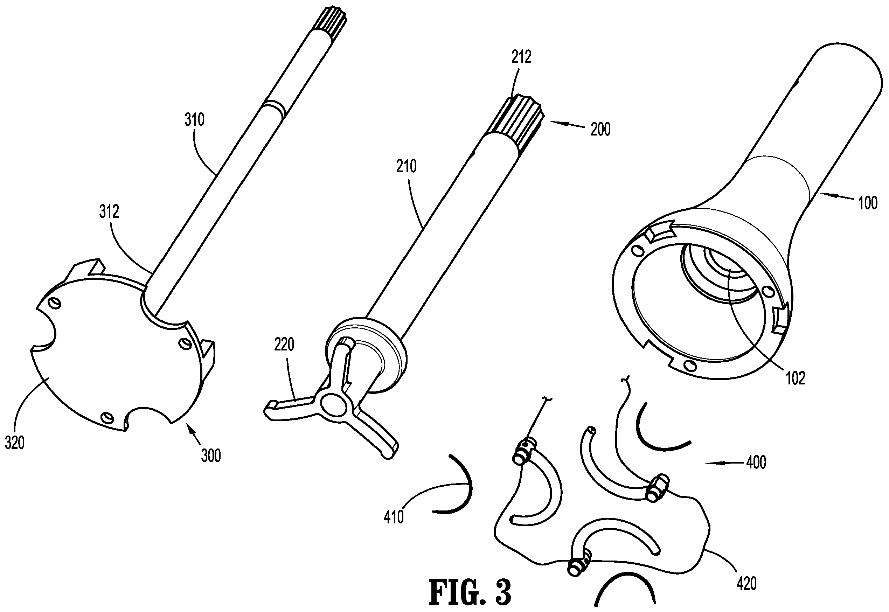

FIGS. 2 and 3 illustrate the outer sleeve 100 defining the lumen 102 therethrough. The lumen 102 extends between distal and proximal end portions 104, 106. The distal and proximal end portions 104, 106 define distal and proximal apertures 104a, 106a, respectively. In particular, the outer sleeve 100 is tapered along a length thereof. For example, the outer sleeve 100 may have a frusto-conical shape. The distal end portion 104 has a larger diameter than a diameter of the proximal end portion 106. The distal end portion 104 is configured to support the needle assembly 400, as will be discussed.

FIGS. 2 and 3 further illustrate the needle driver 200 extending through the lumen 102 of the outer sleeve 100. The needle driver 200 is rotatably supported with the outer sleeve 100. In particular, the needle driver 200 includes an elongate stem 210 and a plurality of wings 220 extending radially outward from a distal end portion 210a of the elongate stem 210. For example, the plurality of wings 220 is disposed adjacent the distal aperture 104a of the outer sleeve 100. A proximal end portion 212 of the elongate stem 210 of the needle driver 200 extends through the proximal aperture 106a of the outer sleeve 100. Under such a configuration, a clinician may manipulate, e.g., rotate, the proximal end potion 212 of the needle driver 200 relative to the outer sleeve 100. In addition, the plurality of wings 220 may be flush with a peripheral portion of the distal end portion 104 of the outer sleeve 100 to facilitate insertion of the suture device 10 into a surgical site. Furthermore, the wings 220 may be uniformly spaced apart about the elongate stem 210. The needle driver 200 defines a passage thererethrough.

The inner shaft 300 is configured to extend through the passage of the needle driver 200. In this manner, the outer sleeve 100, the needle driver 200, and the inner shaft 300 may be concentrically disposed. In particular, the inner shaft 300 includes an elongate member 310 and a support 320 coupled to a distal end portion 312 of the elongate member 310. The support 320 has a planar surface configured to support a plurality of needles 410 of the needle assembly 400 against a distal end portion 104 of the outer sleeve 100. In particular, the support 320 may include guide protrusions 322 configured to be received in respective grooves (not shown) defined in an annular wall of the distal end portion 104 of the outer sleeve 100. Axial mating of the guide protrusions 322 with the respective grooves in the annular wall of the distal end portion 104 facilitates axial displacement of the inner shaft 300 towards and away from the outer sleeve 100.

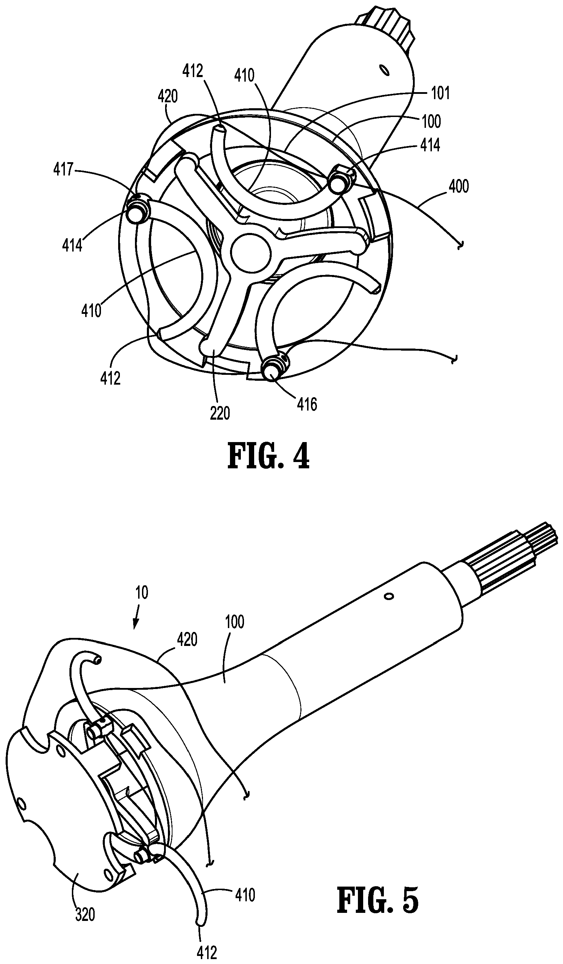

FIG. 4 illustrates the needle assembly 400 including a plurality of needles 410 and a suture 420. The needles 410 are interposed between the support 320 of the inner shaft 300 and the outer sleeve 100. Each needle 410 may include an arcuate profile. Each needle 410 includes a needle tip 412 configured to penetrate through tissue, and a supporting portion 414 including a boss 416 protruding distally to be received in a groove or a bore (not shown) defined in the support 320 (FIG. 3) of the inner shaft 300 such that the needle 410 is rotatable about the boss 416. Alternatively, the boss 416 may further protrude proximally from the supporting portion 414 to be received in a groove or a bore (not shown) defined in, e.g., an annular wall 101, of the outer sleeve 100. In addition, the supporting portion 414 defines a bore 417 dimensioned to receive the suture 420 therethrough. In this manner, the needles 410 are pivotably supported between the support 320 of the inner shaft 300 and the outer sleeve 100 by securing the bosses 416 in the respective grooves or bores of the support 320 and/or the annular wall 101 of the outer sleeve 100.

The plurality of needles 410 is operatively associated with the needle driver 200. The needles 410 are separated by the plurality of wings 220 of the needle driver 200. Under such a configuration, when the wings 220 are rotated about the elongate stem 210, the wings 220 rotate the needles 410 about the respective bosses 416. In this manner, the needles 410 may be transitioned from a retracted position (FIG. 4) to an extended position (FIG. 5). In particular, when the needles 410 are in the retracted position, the needles are flush or, alternatively, radially inward of a peripheral portion of the outer sleeve 100. When the needles 410 are in the extended position, the needles 410 rotate to extend radially outward such that the needles 410 engage and pierce through tissue to form a purse string suture.

The needles 410 may be made from semi-stiff implantable wire. Alternatively, the suture device 10 may include plastic or absorbable materials. Examples of materials that can be used in constructing the needles 410 may include titanium, titanium alloys, stainless steel, nickel, chrome alloys and any other biocompatible implantable metals. Alternatively, other options for materials are liquid crystal polymers, HDPE, polyglycolic acid, and polyglycolid hydroxgacetic acid. At least a portion of each of the needles 410 may be coated with a biocompatible lubricious material that provides for easier delivery of the needles 410 into tissue.

FIGS. 4 and 5 illustrate transition of the suture device 10 from the retracted position to the extended position. Prior to use, the needle assembly 400 is loaded on the support 320 (FIG. 3) of the inner shaft 300 and ultimately secured between the support 320 and the outer sleeve 100. In use, the suture device 10 is positioned adjacent an opening in tissue. FIG. 1 illustrates the suture device 10 having the needle assembly 400 loaded therein. The proximal end portions of the needle driver 200 and the inner shaft 300 extend through the proximal aperture 106a of the outer sleeve 100. The elongate stem 210 of the needle driver 200 is rotated to transition the suture device 10 from the retracted position (FIG. 4) to the extended position (FIG. 5) during which the needles 410 penetrate through tissue. Full rotation of the needle driver 200 enables each needle 410 to pierce through tissue and provide a loop of the suture 420 anchored to tissue. At this time, the needles 410 surround the opening in tissue in order to provide stable anchoring when the loops of the suture 180 are pulled to close the opening. Once the plurality of needles 410 are anchored to a peripheral portion of the opening, the loops of the suture 180 are pulled by the clinician to close the opening. In this manner, the formation of a purse string suture is simplified by, e.g., eliminating the need for maneuvering a needle inside an anal canal, which may reduce injuries to tissue. In addition, the simple configuration of the suture device 10 facilitates disassembly, which, in turn, facilitates reprocessing and/or sterilization of the suture device 10.

Although the illustrative embodiments of the disclosure have been described herein with reference to the accompanying drawings, the above description, disclosure, and figures should not be construed as limiting, but merely as exemplifications of particular embodiments. For example, it is also contemplated that the suture 420 may include barbs or a sharp point to enhance anchoring characteristics thereof. It is further contemplated that the suture device 10 may be adapted for use in robotic surgery.

It is also to be appreciated that the disclosure may be utilized in a number of applications including ligating tissue, hernia mesh repair, and in conjunction with implant drug delivery systems or procedures involving positioning of surgical or implantable devices in patients. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-
1. A suture device comprising:
   an outer sleeve defining a lumen therethrough;
   a needle driver including a stem extending through the lumen of the outer sleeve, and a wing extending radially outward from the stem;
   an inner shaft including an elongate member and a support coupled to a distal end portion of the elongate member; and
   a needle assembly including a needle and a suture, the needle rotatably supported on the support of the inner shaft such that the needle is transitionble between a retracted position, in which, the needle is disposed radially inward of a peripheral portion of the outer sleeve, and an extended position, in which, the needle extends radially outward from the peripheral portion of the outer sleeve.
2. The suture device according to paragraph 1, wherein the outer sleeve has a frusto-conical configuration.
3. The suture device according to paragraph 1, wherein the needle has an arcuate profile.
4. The suture device according to paragraph 1, wherein the needle has a first end having a tip configured to penetrate tissue, and a second end operatively supported on the support of the inner shaft to enable rotation thereof.
5. The suture device according to paragraph 4, wherein the second end of the needle has a boss configured to be received in a groove defined in the support of the inner shaft.
6. The suture device according to paragraph 4, wherein the second end of the needle defines a bore dimensioned to receive the suture therethrough.
7. The suture device according to paragraph 4, wherein the support defines the groove adjacent a peripheral portion of the support.
8. The suture device according to paragraph 1, wherein the outer sleeve defines an aperture at a proximal end portion thereof, the aperture in communication with the lumen of the outer sleeve.
9. The suture device according to paragraph 8, wherein the support of the inner shaft includes a guide protrusion configured to be received in a groove defined in the outer sleeve to facilitate axial displacement of the inner shaft relative to the outer sleeve.
10. The suture device according to paragraph 1, wherein the outer sleeve, the needle driver, and the inner shaft are concentrically disposed.
11. The suture device according to paragraph 1, wherein a proximal end portion of the needle driver extends proximally from a proximal end portion of the outer sleeve, and a proximal end portion of the inner shaft extends proximally from the proximal end portion of the needle driver.
12. A suture device comprising:
   an outer sleeve defining a lumen therethrough;
   a needle driver including a stem and wings extending radially outward from the stem;
   a suture assembly including needles and a suture; and
   an inner shaft including a support configured to support the needles thereon, wherein the needles are separated by the wings of the needle driver and rotatably supported on the support of the inner shaft, such that rotation of the needle driver transitions the needles from a retracted position, in which, the needles are disposed flush with a peripheral portion of the outer sleeve or radially inward of the peripheral portion of the outer sleeve, and an extended position, in which, the needles extend radially outward from the peripheral portion of the outer sleeve to enable penetration through tissue.
13. The suture device according to paragraph 12, wherein each needle includes an arcuate profile having first and second ends.
14. The suture device according to paragraph 13, wherein the first end of the needle includes a tissue penetrating tip, and a second end includes a boss configured to be received in a groove defined in a peripheral portion of the support of the inner shaft for rotation about the boss.
15. The suture device according to paragraph 12, wherein the outer sleeve has a tapered configuration.
16. The suture device according to paragraph 12, wherein the wings of the needle driver are evenly spaced apart.
17. The suture device according to paragraph 12, wherein the outer sleeve and the inner shaft include complementary configurations to facilitate axial displacement of the inner shaft relative to the outer sleeve.
18. A suture device comprising:
   a suture assembly including needles and a suture extending through needles;
   an outer sleeve;
   a needle driver including a stem and wings configured for concomitant rotation with the stem; and
   an inner shaft including a support configured to support the needles of the suture assembly between the support and the outer sleeve, wherein the needle driver is rotatable to transition the needles between a retracted position, in which, the needles are disposed radially inward of the support of the inner shaft and the outer sleeve, and an extended position, in which, the needles are disposed radially outward of the support of the inner shaft and the outer sleeve to engage tissue, wherein the inner shaft is movable towards and away from the outer sleeve to receive the needles therebetween.
19. The suture device according to paragraph 18, wherein the outer sleeve, the needle driver, and the inner shaft are concentrically arranged.
20. The suture device according to paragraph 18, wherein the proximal end portions of the needle driver and the inner shaft extend proximally from the outer sleeve.

## Claims

1. A suture device comprising:
an outer sleeve defining a lumen therethrough;
a needle driver including a stem extending through the lumen of the outer sleeve, and a wing extending radially outward from the stem;
an inner shaft including an elongate member and a support coupled to a distal end portion of the elongate member; and
a needle assembly including a needle and a suture, the needle rotatably supported on the support of the inner shaft such that the needle is transitionble between a retracted position, in which, the needle is disposed radially inward of a peripheral portion of the outer sleeve, and an extended position, in which, the needle extends radially outward from the peripheral portion of the outer sleeve.

2. The suture device according to claim 1, wherein the outer sleeve has a frusto-conical configuration.

3. The suture device according to claim 1 or claim 2, wherein the needle has an arcuate profile.

4. The suture device according to any preceding claim, wherein the needle has a first end having a tip configured to penetrate tissue, and a second end operatively supported on the support of the inner shaft to enable rotation thereof.

5. The suture device according to claim 4, wherein the second end of the needle has a boss configured to be received in a groove defined in the support of the inner shaft; and/or wherein the second end of the needle defines a bore dimensioned to receive the suture therethrough.

6. The suture device according to claim 5, wherein the support defines the groove adjacent a peripheral portion of the support.

7. The suture device according to any preceding claim, wherein the outer sleeve defines an aperture at a proximal end portion thereof, the aperture in communication with the lumen of the outer sleeve; preferably wherein the support of the inner shaft includes a guide protrusion configured to be received in a groove defined in the outer sleeve to facilitate axial displacement of the inner shaft relative to the outer sleeve.

8. The suture device according to any preceding claim, wherein the outer sleeve, the needle driver, and the inner shaft are concentrically disposed; and/or wherein a proximal end portion of the needle driver extends proximally from a proximal end portion of the outer sleeve, and a proximal end portion of the inner shaft extends proximally from the proximal end portion of the needle driver.

9. A suture device comprising:
an outer sleeve defining a lumen therethrough;
a needle driver including a stem and wings extending radially outward from the stem;
a suture assembly including needles and a suture; and
an inner shaft including a support configured to support the needles thereon, wherein the needles are separated by the wings of the needle driver and rotatably supported on the support of the inner shaft, such that rotation of the needle driver transitions the needles from a retracted position, in which, the needles are disposed flush with a peripheral portion of the outer sleeve or radially inward of the peripheral portion of the outer sleeve, and an extended position, in which, the needles extend radially outward from the peripheral portion of the outer sleeve to enable penetration through tissue.

10. The suture device according to claim 9, wherein each needle includes an arcuate profile having first and second ends.

11. The suture device according to claim 9 or claim 10, wherein the first end of the needle includes a tissue penetrating tip, and a second end includes a boss configured to be received in a groove defined in a peripheral portion of the support of the inner shaft for rotation about the boss; and/or wherein the outer sleeve has a tapered configuration.

12. The suture device according to any of claims 9 to 11, wherein the wings of the needle driver are evenly spaced apart.

13. The suture device according to any of claims 9 to 12, wherein the outer sleeve and the inner shaft include complementary configurations to facilitate axial displacement of the inner shaft relative to the outer sleeve.

14. A suture device comprising:
a suture assembly including needles and a suture extending through needles;
an outer sleeve;
a needle driver including a stem and wings configured for concomitant rotation with the stem; and
an inner shaft including a support configured to support the needles of the suture assembly between the support and the outer sleeve, wherein the needle driver is rotatable to transition the needles between a retracted position, in which, the needles are disposed radially inward of the support of the inner shaft and the outer sleeve, and an extended position, in which, the needles are disposed radially outward of the support of the inner shaft and the outer sleeve to engage tissue, wherein the inner shaft is movable towards and away from the outer sleeve to receive the needles therebetween.

15. The suture device according to claim 14, wherein the outer sleeve, the needle driver, and the inner shaft are concentrically arranged preferably wherein the proximal end portions of the needle driver and the inner shaft extend proximally from the outer sleeve.
